# EUROPEAN PATENT SPECIFICATION

(11) **EP 3 274 491 B1**
(45) Date of publication and mention of the grant of the patent: **19.06.2019**
(21) Application number: 16718721.0
(22) Date of filing: 19.03.2016
(51) Int. Cl.: D01D 5/00

(54) **DEVICE AND METHOD TO PRODUCE NANOFIBERS**
VORRICHTUNG UND VERFAHREN ZUR HERSTELLUNG VON NANOFASERN
DISPOSITIF ET PROCÉDÉ POUR PRODUIRE DES NANOFIBRES

(30) Priority: 24.03.2015 FI 20150087
(43) Date of publication of application: 31.01.2018
(73) Proprietor: Helsingin Yliopisto, 00014 Helsingin Yliopisto (FI)
(72) Inventor: LAIDMÄE, Ivo, 61601 Tartumaa (EE); NIEMINEN, Heikki, 00170 Helsinki (FI); SALMI, Ari, 00640 Helsinki (FI); PAULIN, Tor, 00500 Helsinki (FI); RAUHALA, Timo, 00140 Helsinki (FI); FALCK, Kai, 00140 Helsinki (FI); YLIRUUSI, Jouko, 01450 Vantaa (FI); HEINÄMÄKI, Jyrki, 51013 Tartu (EE); HAEGGSTRÖM, Edward, 00140 Helsinki (FI)
(74) Representative: Finnpatent Oy
(86) International application number: PCT/FI2016/050170
(87) International publication number: WO 2016/151191

(56) References cited:
- WO-A2-2012/097229
- CN-U- 202 099 422
- CN-U- 203 096 243
- JP-A- 2009 052 171
- JP-A- 2010 090 484
- JP-A- 2010 216 049
- JP-A- 2010 242 251
- US-A1- 2009 061 496
- US-B2- 8 495 969

## Description

### FIELD

The present invention relates to electrospinning devices and methods to produce fibers and constructs thereof, in particular to nozzle-less electrospinning devices wherein the nanofibers are generated by using pulsed and/or bursted ultrasound.

### BACKGROUND

Electrospinning uses an electric potential difference to draw fine, typically micro or nano scale, fibers from a liquid. The process requires no coagulation chemistry or high temperature to produce solid threads from solution. This makes the process particularly suited for production of fibers using large and complex molecules. Electrospinning has been used to produce nanostructures relevant to many applications. They include filter media, composite materials, protective clothing, micro- and optoelectronic devices, photonic crystals, and flexible photocells. Biomedical applications of nanofibers include use in tissue engineering and production of scaffolds, wound dressings, and drug release systems.

A standard electrospinning setup comprises a spinneret, typically a hypodermic needle, connected to a high-voltage (5 kV to 50 kV) direct current power supply, a syringe pump, and a grounded collector. A polymer solution, sol-gel, particulate suspension, or melt is loaded into the syringe, and this liquid is extruded from the needle tip at a constant rate by the syringe pump.

Standard electrospinning has its drawbacks, especially clogging of the syringe needle. Also spinning of highly viscous polymers through a small-bore needle may be problematic. In addition, to achieve high production rate, the use of many needles is required.

To overcome these problems needleless electrospinning methods and devices have been developed. Yarin and Zussman (Polymer, 45, 2004, pp. 2977-2980) disclosed a two-layer system, where the lower layer was a ferromagnetic suspension, and the upper layer was a polymer solution. When the system was subjected to a strong magnetic field, steady vertical spikes of the magnetic suspension protruded from the interlayer interface, as well as from the free surface of the uppermost surface of the polymer layer. When an electric field was applied to the system, the perturbations of the free surface became sites of jetting directed upward, and solidified nanofibers could be deposited on an upper counter-electrode as in an ordinary electrospinning process.

Although the process of Yarin and Zussman solves some of the problems associated with the standard electrospinning technique, it still has few drawbacks. To stabilize the process, a special type of counter electrode was required. Furthermore, the topology or size of the fiber can be modified only by changing traditional electrospinning parameters, such as the nature of the spinning solution by chemical modification, and by changing the voltage. These are typically slow processes.

JP 2009052171A, JP 2010216049A, CN 203096243U and CN 1986913A describe a method to electro-spin fibers from polymer surface by applying ultrasound to generate waves to the surface of a charged medium. By applying a voltage to the polymer, fibers were ejected from the crests of the waves generated on the surface.

However, electrospinning from a polymer surface without proper focusing or beam steering is a statistical process that provides no spatio-temporal control over ejected fiber. JP 2010216049A suggests various ways to improve focusing the ultrasound, including the use of parallelogram shaped containment vessels, adjusting the number, main direction, frequencies, and amplitudes of the ultrasound source, and by reflecting the ultrasound towards a focal point. However, especially the controlled formation of single fibers or controlled formation of plurality of fibers is still a challenge using relatively low electric fields.

### SUMMARY

The present invention is based on the observation the problems related to controlled formation of polymer fibers can be solved or at least alleviated when the electrospinning is performed by using pulsed and/or bursted ultrasound.

Accordingly, it is an object of this invention to provide a device for producing polymer fibers, the device including:
- an open chamber for a polymer medium,
- voltage generating means comprising an electrode positioned in the open chamber, the voltage generating means configured to apply a voltage to the polymer medium,
- means configured to generate an ultrasound beam, the means comprising an ultrasound signal generating means configured to generate an ultrasound beam driving signal comprising at least one of: a pulse, a burst
- an ultrasound transducer,
- an electrically isolating but acoustically conducting membrane between the open chamber and the ultrasound transducer, and
- a sealed chamber between the membrane and the ultrasound generating means, the chamber comprising an electrically isolating but acoustically conducting material.

It is another object of the present invention is to provide a method for producing polymer fibers using an electrospinning device, the method comprising:
- providing a polymer medium,
- subjecting the polymer medium to an ultrasound beam, and
- applying a voltage to the polymer medium.

According to the method of the present invention, the subjecting is performed by using an ultrasound beam comprising at least one of: a pulse, a burst, and wherein the subjecting is through a sealed chamber comprising electrically isolating but acoustically conducting material, and through electrically isolating but acoustically conducting membrane.

It is still another object of the present invention to provide a method for producing polymer fiber bundle using an electrospinning device comprising a collector plate, the method comprising
- providing a polymer medium,
- subjecting the polymer medium to a plurality of ultrasound beams comprising at least one of: a pulse, a burst, wherein the subjecting is through a sealed chamber comprising electrically isolating but acoustically conducting material, and through electrically isolating but acoustically conducting membrane,
- applying a voltage to the polymer medium,
- guiding the plurality of polymer fibers to a substantially same location on the collector plate, and
- rotating the collecting plate.

Further objects of the present invention are described in the accompanying dependent claims.

Exemplifying and non-limiting embodiments of the invention, both as to constructions and to methods of operation, together with additional objects and advantages thereof, are best understood from the following description of specific exemplifying embodiments when read in connection with the accompanying drawings.

The verbs "to comprise" and "to include" are used in this document as open limitations that neither exclude nor require the existence of un-recited features. The features recited in the accompanied depending claims are mutually freely combinable unless otherwise explicitly stated. Furthermore, it is to be understood that the use of "a" or "an", i.e. a singular form, throughout this document does not exclude a plurality.

### BRIEF DESCRIPTION OF DRAWINGS

Figure 1A shows non-limiting exemplary device for producing polymer fibers according to the present invention.
Figure 1B shows a block of the device depicted in figure 1A.
Figure 2 shows high-speed camera photographs (footage acquired at 238k FPS) of the ultrasound burst -induced fiber formation from an ultrasonically generated polymer protrusion obtained by using an exemplary device according the present invention. (A) = ultrasonic protrusion generated prior to fiber formation. (B) = the protrusion tip at the moment when an ultrasound burst (burst duration 46 µs) hits the tip of the protrusion. (C) = the formation of the Taylor cone (polymer charge -20 kV) from the protrusion tip and (D) = the fiber formed from the Taylor cone.

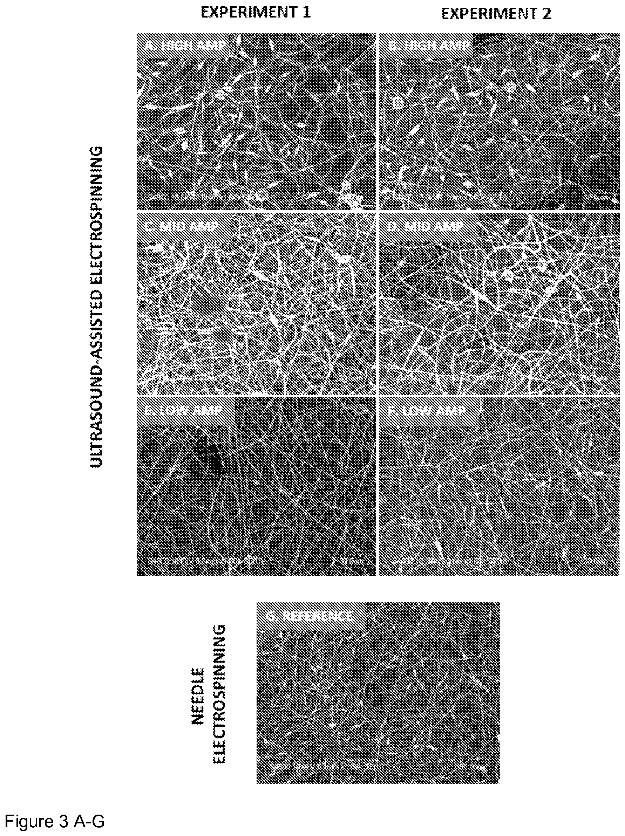

Figure 3 shows exemplary nanofibers produced according to the present invention using various setups (A-F), and nanofibers produced according to prior art (G) (SEM images of nanofibers spun from 3% PEO aqueous solution using high-intensity focused ultrasound with three different acoustic field strengths. The fibers were spatially modified by changing the amplitude of the ultrasound field. Low amp - 260 mV (+ amplification by power amplifier), Mid amp - 500 mV, High amp - 650 mV. Distance from polymer surface to collector - 150 mm, DC voltage - 11.3 kV. The ultrasound column was generated by applying 250 cycle sound bursts at 2.2 MHz carrier frequency and 150 Hz pulse repetition frequency).

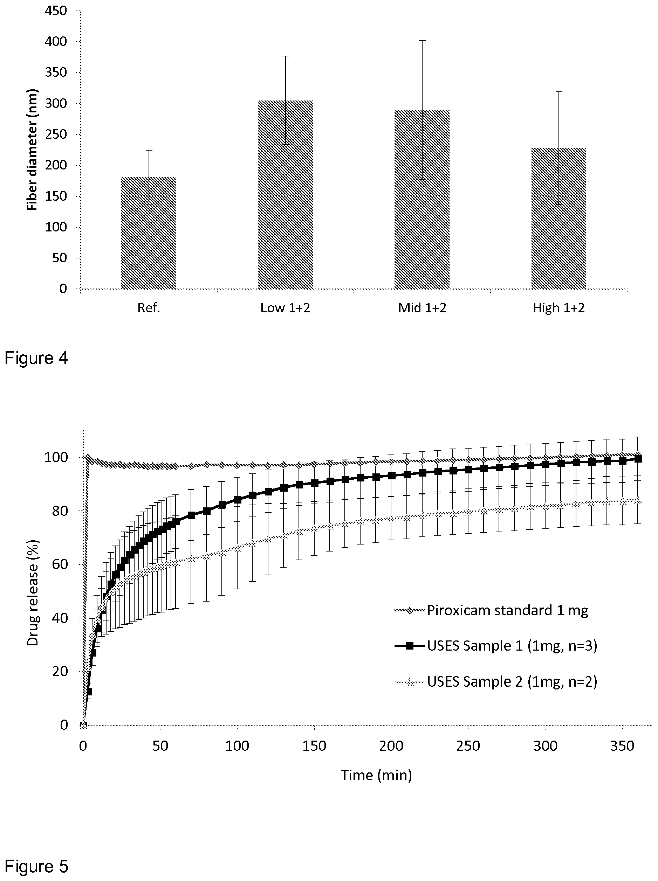

Figure 4 shows thickness of exemplary nanofibers prepared according to the present invention using different ultrasound settings, and thickness of a nanofibers produced according to prior art (Average nanofiber diameters (nm, ±SD) measured in samples prepared by traditional needle ES (Ref.) and amplitude modified ultrasound ES from 3% PEO aqueous solution with three different acoustic field strengths. Low 1+2 - 260 mV (+ amplification by power amplifier), Test 1 + Test 2; Mid 1+2 - 500 mV, Test 1 + Test 2; High 1+2 - 650 mV, Test 1 + Test 2. Distance from polymer surface to collector - 150 mm, DC voltage - 11.3 kV. The ultrasound column was generated by applying 250 cycle sound bursts at 2.2 MHz carrier frequency and 150 Hz pulse repetition frequency.
Figure 5 demonstrates drug release profiles from the polymer fibers prepared according to the present invention (Dissolution of piroxicam from Chitosan/Polyethylene oxide nanomats in water. Samples were prepared by amplitude modified ultrasound ES from Chitosan/polyethylene oxide/piroxicam solution with two different acoustic field strengths. USES Sample 1 - 160 mV (+ amplification by power amplifier), USES Sample 2 - 250 mV. Distance from polymer surface to collector - 340 mm, DC voltage - 21 kV. The ultrasound column was generated by applying sound bursts at 2.2 MHz).
Figure 6 shows images of the polymer solution without an electric field or an acoustic agitation (A), with acoustic agitation (B) and with both electric field and acoustic agitation (C).
Figure 7 shows exemplary fiber and fiber constructs obtainable with the presented technique.
Figure 8 shows an exemplary system for preparing a wound dressing using the method and the device of the present invention.

### DESCRIPTION

As defined herein, *Taylor cone* is a conical symmetric or asymmetric protrusion, e.g. from a polymer bath induced by ultrasound and an electric field.

As defined herein, *a nanofiber* is a fiber-like structure emerging from the Taylor cone with a diameter less than 1000 nm.

As defined herein, *spraying* is producing short segments of nanofibers using ultrasound.

As defined herein, *electrically isolating material* is a material that prevents an electrical short between the ultrasonic transducer and the electrospinner's electrode. The electrically conductive material has conductivity preferably below 10⁻⁸ S/m.

As defined herein, *acoustically conducting material* is a material that permits the employed ultrasound passage without significant attenuation. The acoustically conducting material has attenuation preferably below 10 dB/cm.

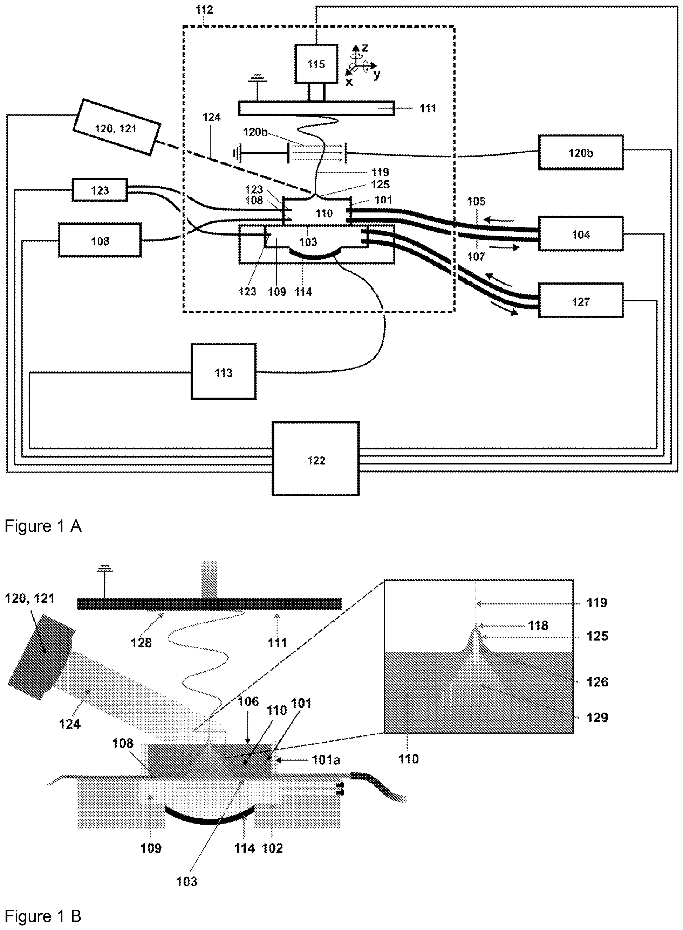

According to one embodiment the present invention concerns an electrospinning device. A device according to a non-limiting exemplary embodiment is shown in figures 1A and 1B. The exemplary electrospinning device includes
- an open chamber (101) for a polymer medium (110), the open chamber including a side wall (101a), and preferably means (104) to supply and/or circulate the polymer medium to the open chamber through an inlet channel (105) and an outlet channel (107),
- means configured to generate an ultrasound beam (129), the means including an ultrasound signal generating means (113) and an ultrasound transducing means, such as ultrasound transducer (114),
- voltage generating means including an electrode (108) positioned in the open chamber, the voltage generating means configured to apply a voltage to the polymer medium, and
- an electrically isolating but acoustically conducting membrane between the open chamber and the ultrasound transducer.

The ultrasound traducer is located so that the ultrasound beam subjects the polymer medium through the electrically isolating membrane.

According to the invention, the ultrasound signal generating means of the device is configured to produce an ultrasound beam comprising at least a pulse or a burst. The presence of continuous waves is not excluded. When the device is in operation, the ultrasound beam that subjects the polymer medium may include one or more burst, one or more pulses, and a time window including continuous ultrasound waves.

The ultrasound beam is configured to generate a protrusion (125) of the polymer from the polymer surface (106).

The voltage generating means configured to apply a voltage to the protrusion (125) so that a Taylor cone (118) is formed from the protrusion, and the polymer fiber (119) is ejected from tip of the Taylor cone.

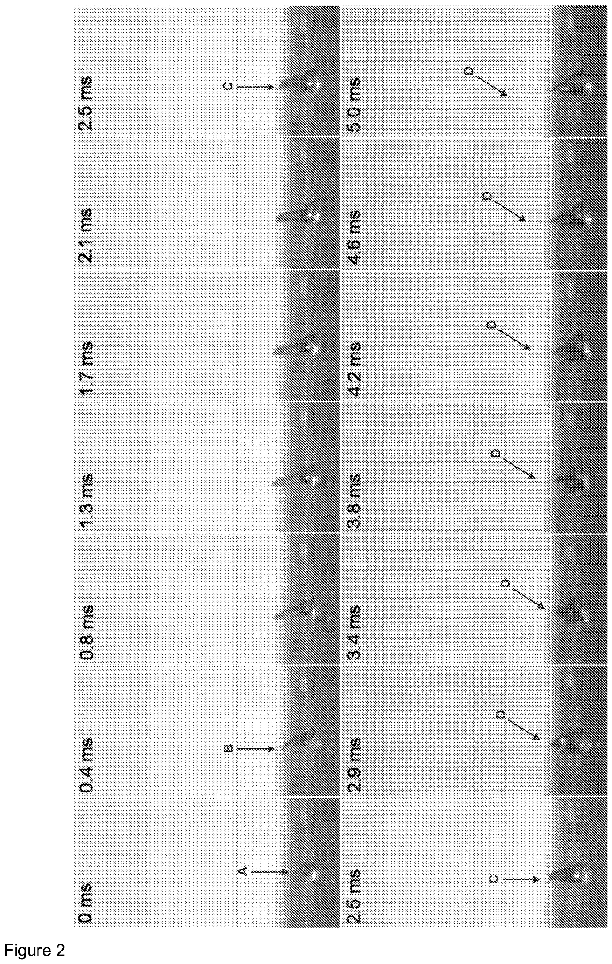

Figure 2 demonstrates the ultrasound burst -induced fiber formation from an ultrasonically generated polymer protrusion of a polymer medium generated by an exemplary device according to the present invention (high-speed camera photographs; footage acquired at 238k FPS). In the figure, figure legend (A) indicates ultrasonic protrusion generated prior to the specific fiber formation (i.e. the protrusion shown therein is the result of preceding focused ultrasound exposure), figure legend (B) indicates the protrusion tip at the moment when an ultrasound burst (burst duration 46 µs) hits the tip of the protrusion, figure legend (C) indicates the formation of the Taylor cone (polymer charge -20 kV) from the protrusion tip, and figure legend (D) indicates the fiber formed from the Taylor cone. By contrast, by using solely continuous ultrasound, the charge used in the experiment shown in figure 2 is not able to initiate fiber formation with the relatively low voltage -20 kV and similar distance from collector to the ultrasound-induced protrusion. The use of higher charge in turn, limits the control of fiber formation by generating violent polymer splashes degrading the produced fiber matrix.

The main function of the membrane (103) is to separate the polymer medium and the ultrasound transducer. The membrane should be substantially electrically isolating to prevent electrical breakdowns, e.g. short circuits. An exemplary membrane is a Mylar membrane, i.e. a polyethylene terephthalate (PET) film.

According to a preferable embodiment the device comprises a sealed chamber (102) filled with an electrically isolating but acoustically conducting material (109) positioned between the membrane and the ultrasound transducer. The material in the sealed chamber (102) should be substantially electrically isolating to prevent electrical breakdowns, e.g. short circuits. In addition, the material should be acoustically conducting (low ultrasound absorption and scattering). When the membrane separating sealed chamber and polymer is flat and thick, the speed of sound in the electrically isolating and acoustically conducting material should be close to that of polymer medium, in order to avoid defocusing the ultrasound beam. Alternatively, the membrane could be curved concentrically with focusing ultrasound transducer. In the latter case, the speed of sound in the materials between the polymer and the transducer need not to be similar to that in polymer. An example of a suitable electrically isolating and acoustically conducting material with speed of sound close to many aqueous polymers is mineral oil. Another example of a suitable material is solid epoxy polymer. Sealing, in turn, prevents the material from moving and prevents air bubbles from forming inside the material.

When the sealed chamber is present, the ultrasound beam is configured to subject the polymer medium through the material in the sealed chamber and the membrane.

According to one embodiment the device includes means (127) configured to circulate and/or to change the material (109) in the sealed chamber (102). Exemplary means include two or more tubes a pump. The advantage of this embodiment is that when the material is liquid, it can be easily changed if needed, e.g. when the properties of the polymer medium is changed. The means (127) can also include means for heating and/or cooling the material.

For control over single fiber properties (controlled diameter, topology, length, porosity and morphology), tight geometrical focusing and a spatio-temporally controlled (stable or dynamic) ultrasound beam (e.g. single frequency, frequency mixing, higher/sub-harmonic generation, shock-wave generation, bursted or continuous wave) can be applied. The beam is required to reach the polymer medium and to generate protrusion preferably by at least one of acoustic radiation force, acoustic streaming, cavitation or physico-chemical modification of polymer (e.g. surface tension, polymer state in polymer medium, viscosity, rheological state, temperature, density, and pressure). Tight focusing of the beam is preferable to generate a narrow (tip diameter <1mm) and high (height >2 mm) protrusion, which is a necessity to generate a Taylor cone and fiber ejection at rather low electric fields, i.e. δ 1 kV/cm. Bursted ultrasound beams with low repetition frequency of 100-300 Hz are preferred to initiate fiber ejection at electric fields δ 1 kV/cm.

Preferably, the device includes an electrically grounded or charged collector plate (111), and more preferably also a collector plate manipulating means (115) configured to move the collector plate in the orthogonal x, y, and z directions. An exemplary motions are translation and rotation.

The collector can be any grounded or charged collector. The collector shape may differ from that of a disk or a plate, and can be e.g. conical or wedge shaped. It may also be stationary, translating, bobbing, wobbling, or rotating along/around one or many axes by a collector manipulating means (115), such as an actuator, e.g. spindle or bobbin. The collector movement may be synchronized with the ultrasonic excitation. The collector surface may be modulated by an acoustic wave, e.g. surface waves, membrane waves, resonances.

The ultrasound generating means is configured to cause a protrusion (125) into the top surface of the polymer medium and thus to form, together with the DC field, a Taylor cone (118). The ultrasound generating means include one or more active transducer elements (114), and one or more signal generators (113), and optionally also phase controllers. According to a preferable embodiment, the ultrasound generating means includes a signal generator (113) configured to produce preferably independent coded signals, i.e. signals whose instantaneous amplitude, phase, and frequency are altered temporally in a predetermined manner, that are driven to the transducer (114). The same technical effect can be obtained using a multiplexer, so that only one signal generator is required. According to another embodiment the ultrasound generating means includes a power amplifier (116) configured to amplify the electronic signal driven to the transducer, preferably through an impedance matching circuit (117) that maximizes the power transfer to the transducer as well as prevents distortion of the drive signal. The power amplifier (116) and the impedance matching circuit (117) are not shown in the drawings.

According to a particular embodiment the device according to the present invention includes a plurality of ultrasound generating means configured to protrude the polymer medium and thus to form a plurality of protrusions on the top surface of the polymer medium, and voltage generating means including an electrode positioned in the open chamber, the voltage generating means configured to apply a voltage to tips of the protrusions of the polymer medium so that in the aid of the voltage generating means, a plurality Taylor cones are formed, and a plurality of polymer fibers are ejected from tip of the Taylor cones. According to this embodiment the device further includes means configured to guide the plurality of polymer fibers to a substantially same location on collector plate that is rotatable.

According to one embodiment the signal generating means includes means that are configured to modify transducer voltage, to modify transducer pulse/burst duration, to modify transducer pulse repetition frequency, to modify transducer frequency content, to modify transducer signal acoustic linearity/non-linearity and/or to modify transducer signal characteristics. Exemplary signal characteristics are sine, sawtooth, square, whiplash, coded signal, noise.

According to one embodiment spinning is done using tone bursts or continuous signals or coded signals with frequencies from kHz to THz, preferably about MHz and most preferably about 2 MHz. Amplitudes can range from a few Pa to GPa, more preferably 100's of kPa to few MPa. Preferentially the amplitude modulation is a 10-500 cycle sinusoidal tone burst with a 0.01-100000 Hz pulse repetition frequency (PRF), most preferentially 100-300 Hz. PRF has an effect on how easily the spinning event starts. PRF of 100-300 Hz and a fountain height of 1-5 mm is preferred to initiate spinning. Continuous wave may be applied after spinning has been initiated.

According to one embodiment the device further includes means for acoustic parametric excitation, i.e. acoustic frequency mixing, or by amplitude modulation, as a way to permit low frequency modulation of a spot close to the Taylor cone tip from inside.

According to one embodiment the device further includes AC/DC generating means (120b) configured to guide the polymer fiber towards the collector plate (111). An exemplary DC generating means is an AC/DC electrode such as a straight conductor.

According to another embodiment the device according to the present invention further includes magnetic, electric field, acoustic and/or optical means configured to externally modulate the tip of the Taylor cone, preferably at the summit of the fountain (126) formed at the focal point. The Taylor cone can be symmetric or asymmetric.

According to a preferable embodiment all functional features of the device, including the sealed chamber (102), the open chamber (101), the membrane (103), the collector (111) and optional collector plate manipulation means (115), and the AC/DC generating means (120b) are located inside a climate chamber (112). This simplifies the control and regulation of physico-chemical properties of the polymer fiber prepared using the device.

According to one embodiment the device according to the present invention includes means (123) configured to control temperature, RH, pH, and atmosphere in order to regulate desired properties of the polymer medium, the material of the sealed chamber, and the atmosphere above the open chamber. According to another embodiment the device further includes means configured to remove air bubbles from the polymer medium and/ or the material in the sealed chamber.

According to another embodiment the device according to the present invention includes imaging means (120) with an optical/acoustic path (124) to the sample to be imagined, configured to imagine the Taylor cone (118) and/or the polymer fiber (119).

According to a preferable embodiment, the imaging means is configured to take single or a sequence of images of the Taylor cone and of at least part of the ejected fiber. According to another embodiment the device includes a plurality of imaging means with different points of view in order to allow producing a 3D image of the Taylor cone and the ejected fiber e.g. by using mirrors or prisms and one imaging sensor. This allows enhanced control of asymmetric vibratory modes and micro vortexes in the Taylor cone. According to one embodiment, a multi-angle view is obtained by using an imaging means including reflecting surfaces and beam splitters. According to another embodiment the imaging means includes a polarizer configured to sharpen the images and to determine internal stress fields in the polymer fiber.

The imaging (single image and sequence of images) can be carried out by any suitable technique capable of obtaining information about the Taylor cone. Examples include but are not limited to optical microscopy, fluorescence microscopy, UV imaging, Raman spectroscopy, interferometry, diffraction and dynamic light scattering. The imaging techniques are known, and suitable imaging devices are commercially available or can in a relatively straight forward manner be customized to present needs. Similarly there exists image capturing and analysis software that can be customized for the present needs. The imaging may be based on direct-, backlight- or self-illumination. The images obtained from the imaging device are stored on an analysis unit, *e.g*. a computer, where they are analyzed using proper software and algorithms in order to determine the shape of the Taylor cone and the properties of the ejecting fiber. The analysis depends on the imaging technique since the origin of the recorded radiation may be different. In case of optical microscopy, each of the images comprises a micrograph or a set of micrographs of the Taylor cone or the ejecting fiber at different stages of the fabrication process. In one embodiment, the image processing comprises determining the size of the projection of the Taylor cone and the nanofiber, nanofibers braids. The processing is based e.g. on cross-sectional surface areas from successive images. The processing also permits determining the presence of splashes based on projection from successive images, and determining of presence of surface waves on the Taylor cone based on successive images.

According to one embodiment the computation of the desired properties of the Taylor cone and ejected fiber is carried out in real time as imaging proceeds based on data obtained until that. The result may be a rough approximate in the beginning and get more accurate as there is more data available (e.g. Bayesian filtering). The data analysis may also be iterative and a 3D reconstruction with topological and morphological information can be made from the image data. According to another embodiment, the computation of the Taylor cone and fiber properties is carried out only after the fabrication process has reached a predefined point, after which the process is terminated or continued. According to another embodiment, the computation of the desired Taylor cone or fiber properties is carried out in a forecasting manner, predicting the future state of the cone and the fiber to allow anticipatory control of the process (for instance, stochastic control to make fibers or fiber braids more even quality or fiber constructs of more even quality). The approach can also be used to make fiber constructs to tighter specifications.

For feedback control of the polymer fiber being produced, the device further includes an optical means, such as a stroboscopic or high-frame rate camera. According to another embodiment the device further includes software and firmware configured to control one of more of the following: level of free polymer surface, shape of the Taylor cone, ejection vector, pointing stability of the ejection vector, presence of polymer splashes, fiber thickness, and material gradients in the fiber or fiber braids, and absence of spinning. Feedback control may or may not use image databases for content-based image retrieval and similarity comparisons.

According to one embodiment, the imaging means is configured to take a single image or a sequence of images of the Taylor cone and at least of part of the elected fiber. According to another embodiment the device includes a plurality of imaging means with different points of view in order to allow producing a 3D image of the Taylor cone and the elected fiber or fiber braid. This allows enhanced control of asymmetric vibratory modes and micro vortexes in the Taylor cone. According to one embodiment, a multi-angle view is obtained by using an imaging means including reflecting surfaces and beam splitters. According to another embodiment the imaging means includes a polarizer configured to sharpen the images and to determine internal stress fields in the polymer fiber.

According to another embodiment the present invention concerns a method for producing polymer fibers using an electrospinning device. An exemplary device suitable for the method includes
- an open chamber (101) including a side wall (101a)
- an ultrasound beam generating means,
- voltage generating means including an electrode positioned in the open chamber, and
- an electrically isolating, but acoustically conducting membrane (103) between the open chamber and the sealed chamber,
the method including steps of:
- providing a polymer medium to the open chamber (101),
- subjecting the polymer medium through the electrically isolating membrane to an ultrasound beam, and
- applying a voltage to the polymer medium.

According to a preferable embodiment, the method includes also collecting the polymer fiber.

According to the method, the ultrasound beam includes at least one of: a peak, a burst.

According to the method of the present invention, the ultrasound burst or pulse generates a protrusion on the top surface of the polymer medium located in the open chamber. It is preferred that the ultrasound burst or pulse is so strong that the tip of the protrusion is above the top edge of the walls of the open chamber. This ensures a stable electric field between the tip of the protrusion and the target by diminishing the effects of the walls on the electric DC-field.

According to the method of the present invention, a voltage is applied to the polymer medium by using an electrode located in the open chamber and within the polymer medium. The voltage is preferably between 5 kV and 50 kV. This forms a Taylor cone and thus ejection of a polymer fiber from the tip of the Taylor cone. According to the method the ejecting polymer fiber is collected onto a grounded or charged collector that is flat, conical or of any other geometric shape. The collectors are known in the art.

The use of ultrasound allows switching between no spinning, spinning, and spraying modes in a rapid intermittent manner. In one implementation, it is characterized by employing a focused piezo ceramic transducer into which a number of sectors, preferably 16, have been engraved to allow phase controlled focused ultrasound. Using the annular phased array construct, the tip angle and symmetry of the Taylor cone can be adjusted dynamically and vorticity can be introduced into the cone to make the extruding fiber helical.

According to a preferable embodiment the Taylor cone, especially the tip of the Taylor cone and/or the laminar part of the top surface of the polymer medium is modulated by an external modulator so that the shape and/or the form of the ejecting polymer fiber is controlled and tuned according to needs. Exemplary modulations are magnetic, electric field, acoustic, thermal, and optical modulation.

According to another embodiment the method according to the present invention further includes guiding the polymer fiber ejected from the tip of the Taylor cone by an external electric DC field and collecting the fiber onto a grounded or charged collector.

According to another embodiment the present disclosure concerns a method for producing polymer fibers using an electrospinning device, the device including. An exemplary device suitable for the method includes
- an open chamber (101),
- an ultrasound beam generating means,
- voltage generating means including an electrode positioned in the open chamber, and
- an electrically isolating, but acoustically conducting membrane (103) between the open chamber and the sealed chamber, and
- a collector plate (111),
the method including steps of:
- providing a polymer medium to the open chamber
- subjecting the polymer medium to plurality of ultrasound beams comprising at least one of: a pulse, burst, to so that a plurality of protrusions on a surface of the polymer medium is formed,
- applying a voltage to the polymer medium so that a plurality of Taylor cones are formed on the protrusions, and polymer fibers are ejected from tip of the Taylor cones,
- guiding the plurality of polymer fibers to a substantially identical location on the collector plate, and
- rotating the collecting plate so that a braid comprising the plurality of polymer fibers is formed.

According to a preferable embodiment the polymer medium is liquid. According to another preferable embodiment the polymer medium includes two or more polymers, one or more solvents, and/or one or more substrates such as chemical or biological entities. The solvent makes the polymer medium less viscous, and helps to incorporate the one or more substrates into the polymer collected construct. Exemplary substrates are drug molecules, prodrug molecules, drug candidate molecules, nanoparticles, gold particles, such as gold nanoparticles, cells, viruses, bisphosphonates, steroids, proteoglycan, collagen, and growth factors. According to another embodiment, the substrate is selected from drug molecules, gold particles, viruses and cells.

Biologically active materials that may be of interest to the technology include analgesics, antagonists, anti-inflammatory agents, anthelmintics, antianginal agents, antiarrhythmic agents, antibiotics (including penicillins), anticholesterols, anticoagulants, anticonvulsants, antidepressants, antidiabetic agents, antiepileptics, antigonadotropins, antihistamines, antihypertensive agents, antimuscarinic agents, antimycobacterial agents, antineoplastic agents, antipsychotic agents, immunosuppressants, antithyroid agents, antiviral agents, antifungal agents, anxiolytic sedatives (hypnotics and neuroleptics), astringents, beta-adrenoceptor blocking agents, blood products and substitutes, anti-cancer agents, cardiacinotropic agents, contrast media, corticosterioids, cough suppressants (expectorants and mucolytics), diuretics, dopaminergics (antiparkinsonian agents), haemostatics, immunosuppressive and immunoactive agents, lipid regulating agents, muscle relaxants, parasympathomimetics, parathyroid calcitonin and biphosphonates, prostaglandins, radiopharmaceuticals, sex hormones (including steroids), anti-allergic agents, stimulants and anorexics, sympathomimetics, thyroid agents, vasidilators, neuron blocking agents, anticholinergic and cholinomimetic agents, antimuscarinic and muscarinic agents, vitamins, and xanthines.

Exemplary medicaments suitable for the present technology are entacapone, esomeprazole, atorvastatin, rabeprazole, piroxicam and olanzapine. An exemplary medicament is piroxicam (4-hydroxy-2-methyl-N-(2-pyridinyl)-2H-1,2-benzothiazine-3-carboxamide 1,1-dioxide).

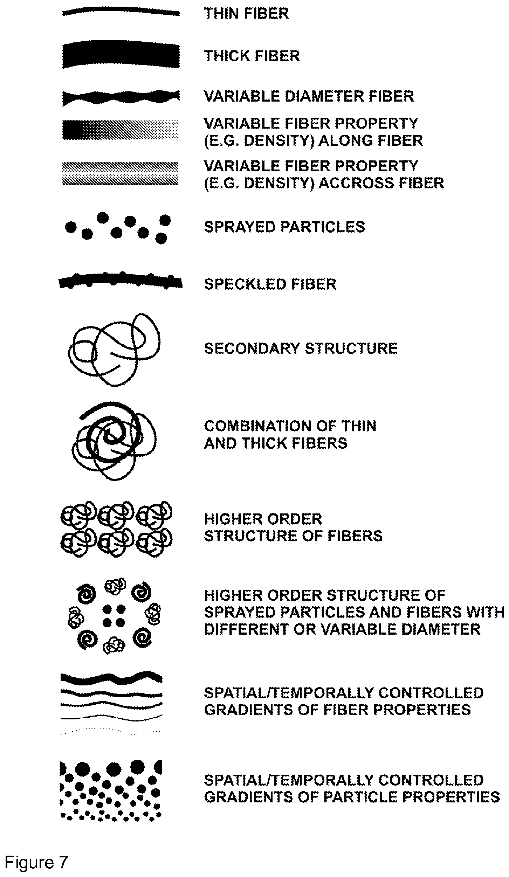

The fiber produced according the present method are typically 5 to 1000 nm in diameter, preferably about 100 nm. Nanofiber braids are larger. Exemplary fibers obtainable by the method of the present invention are shown in figure 7. The fiber produced may include nanoparticles, same or different, as substrates.

The polymer fibers produced according to the method of the present disclosure can be solid or contain inclusions. The fiber can be homogenous or can contain gradients and/or fractal structures. If biological materials such as living cells are incorporated into the polymer fiber, the polymer is selected such that the cells can survive in the polymer fiber. According to another embodiment the polymer fiber is selected such that the fiber changes the properties of the cells as required. The polymer can also be selected such that it is biologically resorbable.

According to another embodiment the method further includes taking one or more images of the Taylor cone and/or the ejected fiber or fiber braid, and storing and/or analyzing the images. Storing the images is useful, especially in pharmaceutical applications where quality control and postproduction verifiability is required. In addition, the analysis allows detection of potential structural defects in the collected construct, and online tuning of fabrication parameters during polymer fiber formation.

Figure 3 shows exemplary nanofibers (A-F) produced using 3% polyethylene oxide (PEO) aqueous solution as polymer medium and high (650 mV, generated by signal generator (113) and then amplified by the power amplifier (116)), medium (500 mV), and low (260 mV) ultrasound amplitude according to the method and the device of the present disclosure. The fiber (G) is produced by using needle spinning instrument according to prior art.

As evident in figure 3A-F, the fiber shape can be tuned as a function of the ultrasound amplitude. The columns represent two parallel experiments demonstrating the repeatability of the method. This is an advantage especially when fibers for medical and pharmaceutical applications are prepared. The mean thickness of the fibers is shown in Figure 3. The results demonstrate that fiber topology were modified by changing the amplitude of the ultrasound field.

As shown by results in Figure 3, by modifying the ultrasonic column (125) height by means of the driving voltage, one can modify the properties, e.g. the mean thickness or thickness variation, of the produced fiber. The voltage across the transducer (114) can be controlled (122) as a function of time with great precision (µs to ms). Therefore, the fiber properties can be modified along the fiber with high resolution. Combining this with external manipulation (115, 120b), one can control the produced fiber construct (128) in time and space, equivalent to 3D printing, but on micro scale. Moreover, the massless "nozzle" size can be controlled in time, which is not possible with nozzle-based 3D printing approaches.

Figure 4 shows thickness of exemplary nanofibers prepared according to the present invention using different ultrasound settings, and thickness of a nanofibers produced according to prior art. The mean values represent the average thickness of fiber samples presented in Figure 3 and SD represents the respective distribution. The result shows that the mean thickness value can be modified with different acoustic field strengths demonstrating a non-chemical modification of the fiber topology. The thickness distributions reflect the topological heterogeneity of the fibers seen in Figure 3.

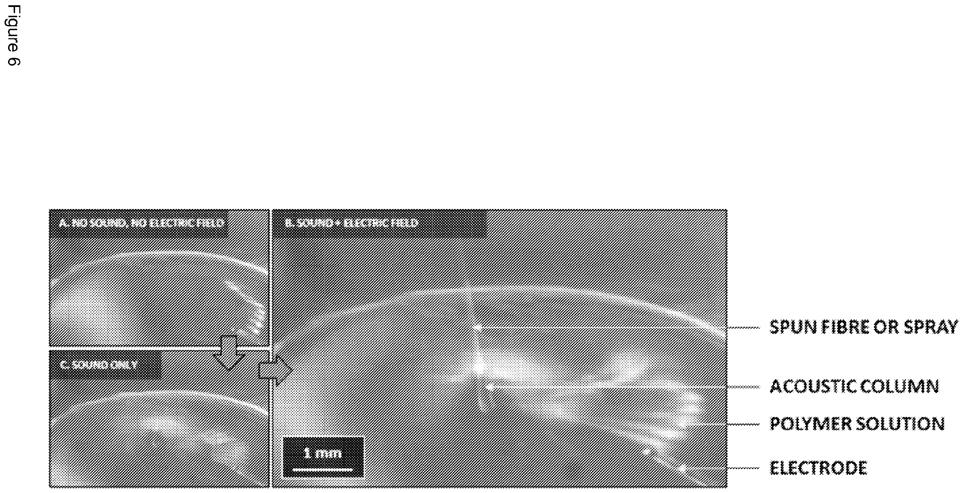

An exemplary image of the top surface of the polymer medium is shown in Figure 6A-C. Figure 6A shows the situation where there polymer medium is neither subjected to ultrasound nor electric field. Figure 6B shows the situation when the polymer medium is subjected to ultrasound and thus a protrusion is formed. In Figure 6C, the polymer medium is subjected to ultrasound and an electric field. As seen, a Taylor cone is formed and polymer fiber is ejected from the tip of the Taylor cone (acoustic fountain).

According to a preferable embodiment a substrate such as a drug molecule is incorporated into the polymer fiber. This is done by using a polymer medium including the substrate such as a drug molecule in the method of the present disclosure.

According to one embodiment, Chitosan/PEO fibers including piroxicam were prepared using the method and device according to the present disclosure. Piroxicam dissolution in water media from the Chitosan/PEO fibers of various diameters is shown in figure 5.

The release profile of substances from the fibers can be altered by e.g. embedding nano bubbles or light absorbing nanoparticles (e.g. gold or light controlled organic molecules) into the fibers and applying external agitation, e.g. by applying sound or light. This release could be triggered by the environment, e.g. pH, enzymes, and temperature for controlled release at a desired site.

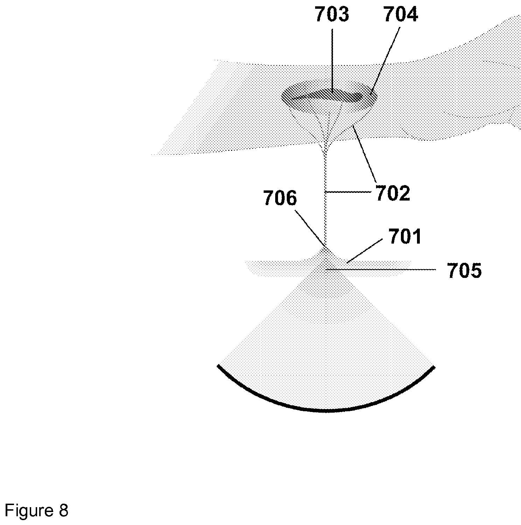

The device and the method of the present invention is suitable for preparing wound dressing and bad-aids. According to a preferable embodiment, the wound dressing (or the band-aid) preferably includes medicament that enhances wound healing. An exemplary system is shown in figure 8. Accordingly, a polymer medium including a desired drug molecule (701) is provided in the open chamber of the device according to the present invention. Ultrasound beam (705) is then allowed to generate a protrusion on surface of the polymer medium, and by applying a voltage to the polymer medium, a Taylor cone (706) is formed from the protrusion, and a polymer fiber construct (702) is ejected from tip of the Taylor cone. The construct is guided to the wound (703) to form a wound dressing (704).

The device can be hand held, and could be used either in hospitals at bedside or by the patient at home or in the field, e.g. home-made wound dress, band-aid, or plaster all with or without drugs.

According to another embodiment the present invention concerns a computer program product including computer executable instructions for controlling a programmable processor to examine the Taylor cone and/or an ejected fiber wherein the program is adapted to evaluate the data obtainable by a method according to the present invention. The computer program product can be implemented with one or more circuits, each of which can be a programmable processor circuit provided with appropriate software, a dedicated hardware processor e.g. an application specific integrated circuit "ASIC", or a configurable hardware processor, e.g. a field programmable gate array "FPGA".

According to another embodiment, the present invention concerns a polymer fiber, comprising a substrate, obtainable by a method using an electrospinning device of the present invention, the method comprising
- providing a polymer medium comprising a substrate
- subjecting a ultrasound beam comprising at least a pulse or a burst to the polymer medium, and
- applying a voltage to the polymer medium,

The substrate is preferably selected from the group consisting of drug molecules, inorganic particles, viruses, cells, and biologically active molecules. Exemplary the inorganic particles are gold particles

According to another embodiment, the substrates are selected from drug molecules, prodrug molecules, drug candidate molecules, nanoparticles, gold particles, such as gold nanoparticles, cells, viruses, bisphosphonates, steroids, proteoglycan, collagen, and growth factors.

According to a particular embodiment the biologically active molecules are selected from a group consisting of proteins, peptides, nucleic acids, oligosaccharides, polysaccharides, lipids, hormone, growth factors, antibodies.

According to a particular embodiment, the peptide comprises 2 to and 300 amino acids, wherein the amino acids are naturally occurring amino acids and/or non-naturally occurring amino acids.

According to a particular embodiment, the antibodies are chimeric, humanized or fully human antibody, and/or an antigen-binding fragment thereof.

According to still another embodiment, the present invention relates to a scaffold comprising a polymer fiber comprising a substrate, obtainable by the method of the present invention.

The non-limiting, specific examples provided in the description given above should not be construed as limiting the scope and/or the applicability of the appended claims.

## Claims

1. A device for producing polymer fiber (119), the device comprising,
- an open chamber (101) for a polymer medium (110),
- voltage generating means comprising an electrode (108) positioned in the open chamber, the voltage generating means configured to apply a voltage to the polymer medium,
- ultrasound beam generating means comprising a signal generating means (113) configured to generate an ultrasound beam driving signal, the signal comprising at least one of: a pulse, a burst
- an ultrasound transducer (114),
- an electrically isolating but acoustically conducting membrane (103) between the open chamber and the ultrasound transducer, **characterized in that** the device comprises a sealed chamber (102) between the membrane (103), and the ultrasound transducer (114), the chamber comprising an electrically isolating but acoustically conducting material (109).

2. The device according to claim 1, wherein the signal generating means (113) further comprises means (116,117) configured to modify one or more of: transducer voltage, transducer pulse/burst duration, transducer pulse repetition frequency, transducer frequency content, transducer signal acoustic linearity/non-linearity and transducer signal characteristics.

3. The device according to claim 1 or 2, wherein the device comprises means (127) configured to circulate and/or to change the material (109) in the sealed chamber (102).

4. The device according any of claims 1 to 3, wherein the material (109) is selected from oil, in particular mineral oil, and solid epoxy polymer.

5. The device according to any of claims 1 to 4, wherein the membrane (103) comprises polyethylene terephthalate.

6. The device according to any of claims 1 to 5, the device further comprising
- an electrically grounded or charged collector plate (111) configured to collect the polymer fiber, and preferably a collector plate manipulating means (115) configured to move the collector plate along orthogonal x, y, and z directions.

7. The device according to claim 6, the device further comprising:
- means (120b) configured to generate an AC/DC field and to guide the polymer fiber towards the collector plate.

8. The device according to any of claims 1 to 7, the device further comprising means (120) configured to take one or more images from a Taylor cone and/or an ejected polymer fiber (119).

9. A method for producing polymer fiber, using an electrospinning device,
the method comprising:
- providing a polymer medium,
- subjecting the polymer medium to an ultrasound beam wherein the ultrasound beam comprises at least one of: a pulse, a burst, and
- applying a voltage to the polymer medium, **characterized in that**
- the subjecting is through a sealed chamber comprising an electrically isolating but acoustically conducting material, and through electrically isolating but acoustically conducting membrane.

10. The method according to claim 9, the method further comprising collecting the polymer fiber onto an electrically grounded or charged collector plate.

11. The method according to claim 9 or 10, the method further comprising focusing an ultrasound beam to a Taylor cone, and varying amplitude, frequency, and/or pulse repetition frequency of the ultrasound beam.

12. The method according to any of claims 9 to 11, the method further comprising guiding the polymer fiber by an external AC/DC field.

13. A method for producing polymer fiber bundle using an electrospinning device comprising a collector plate, the method comprising
- providing a polymer medium,
- subjecting the polymer medium to a plurality of ultrasound beams comprising at least one of: a pulse, a burst,
- applying a voltage to the polymer medium,
- guiding the plurality of polymer fibers to a substantially same location on the collector plate, and
- rotating the collecting plate, **characterized** the subjecting is through a sealed chamber comprising an electrically isolating but acoustically conducting material, and through electrically isolating but acoustically conducting membrane.

14. The method according to claim 13, the polymer medium further comprising a solvent and/or a substrate.

15. The method according to claim 14, wherein the substrate is selected from drug molecules, prodrug molecules, drug candidate molecules, nanoparticles, gold particles, viruses and cells.

## Patentansprüche

1. Vorrichtung zum Herstellen von Polymerfaser (119), wobei die Vorrichtung umfasst:
- eine offene Kammer (101) für ein Polymermedium (110),
- Spannung erzeugendes Mittel, umfassend eine Elektrode (108), die in der offenen Kammer platziert ist, wobei das Spannung erzeugende Mittel ausgestaltet ist, um eine Spannung an das Polymermedium anzulegen,
- Ultraschallstrahl erzeugendes Mittel, umfassend ein Signal erzeugendes Mittel (113), ausgestaltet, um ein Ultraschallstrahl-Steuersignal zu erzeugen, wobei das Signal mindestens eines umfasst von einem Impuls, einem Stoß,
- einen Ultraschallwandler (114),
- eine elektrisch isolierende, jedoch akustisch leitende Membran (103) zwischen der offenen Kammer und dem Ultraschallwandler, **dadurch gekennzeichnet, dass** die Vorrichtung eine versiegelte Kammer (102) zwischen der Membran (103) und dem Ultraschallwandler (114) umfasst, wobei die Kammer ein elektrisch isolierendes, jedoch akustisch leitendes Material (109) umfasst.

2. Vorrichtung nach Anspruch 1, wobei das Signal erzeugende Mittel (113) ferner Mittel (116, 117) umfasst, ausgestaltet, um eines oder mehrere zu modifizieren von: Wandlerspannung, Wandler-Impuls-/Stoßdauer, Wiederholungsfrequenz des Wandlerimpulses, Wandlerfrequenzinhalt, akustischer Linearität/Nichtlinearität des Wandlersignals und Kennzeichen des Wandlersignals.

3. Vorrichtung nach Anspruch 1 oder 2, wobei die Vorrichtung Mittel (127) umfasst, ausgestaltet, um das Material (109) in der versiegelten Kammer (102) zirkulieren zu lassen und/oder zu wechseln.

4. Vorrichtung nach einem von Anspruch 1 bis 3, wobei das Material (109) aus Öl, insbesondere Mineralöl, und festem Epoxidpolymer ausgewählt ist.

5. Vorrichtung nach einem von Anspruch 1 bis 4, wobei die Membran (103) Polyethylenterephthalat umfasst.

6. Vorrichtung nach einem von Anspruch 1 bis 5, wobei die Vorrichtung ferner umfasst:
- eine elektrisch geerdete oder geladene Kollektorplatte (111), ausgestaltet, um die Polymerfaser aufzunehmen, und bevorzugt ein Kollektorplatten-Betätigungsmittel (115), ausgestaltet, um die Kollektorplatte längs orthogonaler x-, y- und z-Richtungen zu bewegen.

7. Vorrichtung nach Anspruch 6, wobei die Vorrichtung ferner umfasst:
- Mittel (120b), ausgestaltet, um ein Wechselstrom/Gleichstrom-Feld zu erzeugen und die Polymerfaser zur Kollektorplatte zu führen.

8. Vorrichtung nach einem von Anspruch 1 bis 7, wobei die Vorrichtung ferner Mittel (120) umfasst, ausgestaltet, um ein oder mehrere Bilder von einem Taylor-Kegel und/oder einer ausgestoßenen Polymerfaser (119) aufzunehmen.

9. Verfahren zum Herstellen von Polymerfaser unter Benutzen einer Elektrospinnvorrichtung,
wobei das Verfahren umfasst:
- Bereitstellen eines Polymermediums,
- Unterwerfen des Polymermediums einem Ultraschallstrahl, wobei der Ultraschallstrahl mindestens eines umfasst von: einem Impuls, einem Stoß, und
- Anlegen einer Spannung an das Polymermedium, **dadurch gekennzeichnet, dass**
- das Unterwerfen durch eine versiegelte Kammer, umfassend ein elektrisch isolierendes, jedoch akustisch leitendes Material, und durch eine elektrisch isolierende, jedoch akustisch leitende Membran erfolgt.

10. Verfahren nach Anspruch 9, wobei das Verfahren ferner das Aufnehmen der Polymerfaser auf einer elektrisch geerdeten oder geladenen Kollektorplatte umfasst.

11. Verfahren nach Anspruch 9 oder 10, wobei das Verfahren ferner das Fokussieren eines Ultraschallstrahls auf einen Taylor-Kegel und das Verändern der Amplitude, der Frequenz und/oder der Impulswiederholungsfrequenz des Ultraschallstrahls umfasst.

12. Verfahren nach einem von Anspruch 9 bis 11, wobei das Verfahren ferner das Führen der Polymerfaser mittels eines äußeren Wechselstrom/Gleichstrom-Feldes umfasst.

13. Verfahren zum Herstellen eines Polymerfaserbündels unter Benutzen einer Elektrospinnvorrichtung, umfassend eine Kollektorplatte, wobei das Verfahren umfasst:
- Bereitstellen eines Polymermediums,
- Unterwerfen des Polymermediums mehreren Ultraschallstrahlen, umfassend mindestens eines von: einem Impuls, einem Stoß,
- Anlegen einer Spannung an das Polymermedium,
- Führen der mehreren Polymerfasern zu einem im Wesentlichen selben Ort auf der Kollektorplatte und
- Rotierenlassen der Kollektorplatte, **dadurch gekennzeichnet, dass** das Unterwerfen durch eine versiegelte Kammer, umfassend ein elektrisch isolierendes, jedoch akustisch leitendes Material, und durch eine elektrisch isolierende, jedoch akustisch leitende Membran erfolgt.

14. Verfahren nach Anspruch 13, wobei das Polymermedium ferner ein Lösemittel und/oder ein Substrat umfasst.

15. Verfahren nach Anspruch 14, wobei das Substrat aus Arzneimittelmolekülen, Prodrug-Molekülen, Arzneimittelkandidat-Molekülen, Nanoteilchen, Goldteilchen, Viren und Zellen ausgewählt wird.

## Revendications

1. Dispositif destiné à la production de fibre de polymère (119), le dispositif comprenant :
- une chambre ouverte (101) destinée à un milieu de polymère (110),
- un moyen de génération d'une tension comprenant une électrode (108) positionnée dans la chambre ouverte, le moyen de génération d'une tension étant conçu pour appliquer une tension au milieu de polymère,
- un moyen de génération d'un faisceau d'ultrasons comprenant un moyen de génération d'un signal (113) conçu pour générer un signal d'entraînement de faisceau d'ultrasons, le signal comprenant au moins un parmi : une impulsion, une rafale
- un transducteur d'ultrasons (114),
- une membrane isolante électrique mais conductrice acoustique (103) située entre la chambre ouverte et le transducteur d'ultrasons, **caractérisé en ce que** le dispositif comprend une chambre étanche (102) située entre la membrane (103) et le transducteur d'ultrasons (114), la chambre comprenant un matériau isolant électrique mais conducteur acoustique (109).

2. Dispositif selon la revendication 1, dans lequel le moyen de génération d'un signal (113) comprend en outre des moyens (116, 117) conçus pour modifier au moins un parmi : une tension de transducteur, une impulsion de transducteur/une durée de rafale, une fréquence de répétition d'impulsions de transducteur, un contenu de fréquence de transducteur, une linéarité/non linéarité acoustique de signal de transducteur et des caractéristiques de signal de transducteur.

3. Dispositif selon la revendication 1 ou la revendication 2, dans lequel le dispositif comprend un moyen (127) conçu pour faire circuler et/ou pour changer le matériau (109) dans la chambre étanche (102) .

4. Dispositif selon l'une quelconque des revendications 1 à 3, dans lequel le matériau (109) est choisi parmi de l'huile, en particulier de l'huile minérale, et un polymère époxy solide.

5. Dispositif selon l'une quelconque des revendications 1 à 4, dans lequel la membrane (103) comprend du téréphtalate de polyéthylène.

6. Dispositif selon l'une quelconque des revendications 1 à 5, le dispositif comprenant en outre
- une plaque de collecteur électriquement mise à la terre ou chargée (111) conçue pour recueillir la fibre de polymère, et préférablement un moyen de manipulation de plaque de collecteur (115) conçu pour déplacer la plaque de collecteur le long des directions orthogonales X, Y et Z.

7. Dispositif selon la revendication 6, le dispositif comprenant en outre :
- un moyen (120b) conçu pour générer un champ CA/CC et pour guider la fibre de polymère en direction de la plaque de collecteur.

8. Dispositif selon l'une quelconque des revendications 1 à 7, le dispositif comprenant en outre un moyen (120) conçu pour prendre au moins une image à partir d'un cône de Taylor et/ou à partir d'une fibre de polymère éjectée (119).

9. Procédé de production de fibre de polymère en utilisant un dispositif d'électrofilage,
le procédé consistant à :
- obtenir un milieu de polymère,
- soumettre le milieu de polymère à un rayon d'ultrasons dans lequel le rayon d'ultrasons comprend au moins un parmi une impulsion, une rafale, et
- appliquer une tension au milieu de polymère, **caractérisé en ce que**
- l'assujettissement se fait à travers une chambre étanche comprenant un matériau isolant électrique mais conducteur acoustique, et à travers une membrane isolante électrique mais conductrice acoustique.

10. Procédé selon la revendication 9, le procédé comprenant en outre la collecte de la fibre de polymère sur une plaque de collecteur électriquement mise à la terre ou chargée.

11. Procédé selon la revendication 9 ou la revendication 10, le procédé comprenant en outre la focalisation d'un faisceau d'ultrasons sur un cône de Taylor, et la variation de l'amplitude, de la fréquence et/ou de la fréquence de répétition des impulsions du faisceau d'ultrasons.

12. Procédé selon l'une quelconque des revendications 9 à 11, le procédé comprenant en outre le guidage de la fibre de polymère par un champ CA/CC externe.

13. Procédé de production d'un faisceau de fibre de polymère en utilisant un dispositif d'électrofilage comprenant une plaque de collecteur, le procédé consistant à :
- obtenir un milieu de polymère,
- soumettre le milieu de polymère à une pluralité de faisceaux d'ultrasons comprenant au moins un parmi une impulsion, une rafale,
- appliquer une tension sur le milieu de polymère,
- guider la pluralité de fibres de polymère vers un emplacement sensiblement identique sur la plaque de collecteur, et
- faire tourner la plaque de collection, **caractérisée en ce que** l'assujettissement se fait à travers une chambre étanche comprenant un matériau isolant électrique mais conducteur acoustique et à travers une membrane isolante électrique mais conductrice plastique.

14. Procédé selon la revendication 13, le milieu de polymère comprenant en outre un solvant et/ou un substrat.

15. Procédé selon la revendication 14, dans lequel le substrat est choisi parmi des molécules de médicament, des molécules de prodrogue, des molécules de candidat médicament, des nanoparticules, des particules d'or, des virus et des cellules.
